# EUROPÄISCHE PATENTSCHRIFT

(11) **EP 4 558 474 B1**
(45) Veröffentlichungstag und Bekanntmachung des Hinweises auf die Patenterteilung: **24.06.2026**
(21) Anmeldenummer: 23722529.7
(22) Anmeldetag: 26.04.2023
(51) Int. Cl.: C07C 45/50, C07C 47/02, B01J 31/00, B01J 31/02, B01J 31/20, B01J 31/22, B01J 31/16, B01J 31/24, B01J 35/50, B01J 35/56, B01J 27/224

(54) **VERFAHREN ZUR HYDROFORMYLIERUNG VON KURZKETTIGEN OLEFINEN IN DER GASPHASE**
METHOD FOR HYDROFORMYLATION OF SHORT-CHAIN OLEFINS IN THE GAS PHASE
PROCÉDÉ D'HYDROFORMYLATION D'OLÉFINES À CHAÎNE COURTE EN PHASE GAZEUSE

(30) Priorität: 19.07.2022 EP 22185749
(43) Veröffentlichungstag der Anmeldung: 28.05.2025
(73) Patentinhaber: Evonik Oxeno GmbH & Co. KG, 45772 Marl (DE)
(72) Erfinder: FRANKE, Robert, 45772 Marl (DE); ARSENJUK, Linda, 44369 Dortmund (DE); NENTWICH, Corina, 45711 Datteln (DE); STENGER, Frank, 63755 Alzenau (DE); KRISTEN, Marc Oliver, 45721 Haltern am See (DE); KREIS, Peter, 44227 Dortmund (DE)
(74) Vertreter: Evonik Patent Association
(86) Internationale Anmeldenummer: PCT/EP2023/060930
(87) Internationale Veröffentlichungsnummer: WO 2024/017514

(56) Entgegenhaltungen:
- EP-A1- 3 632 885
- EP-A1- 3 632 886
- EP-A1- 3 736 258
- EP-A1- 3 744 707

## Beschreibung

Das Projekt, das zu dieser Patentanmeldung führte, wurde im Rahmen der Fördervereinbarung Nr. 869896 aus dem Forschungs- und Innovationsprogramm Horizon 2020 der Europäischen Union gefördert.

Die vorliegende Erfindung betrifft ein Verfahren zur Hydroformylierung von kurzkettigen Olefinen, insbesondere C2- bis C5-Olefinen in einem Reaktor, bei dem das Katalysatorsystem heterogenisiert auf einem Support aus einem porösen keramischen Material vorliegt und wobei bei Verfahrensstandzeiten Synthesegas oder Kohlenmonoxid durch den Reaktor geleitet wird.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Die Probleme bei den bekannten Verfahren sind vielfältig, wobei diese Probleme insbesondere damit verknüpft sind, dass sowohl Rhodium als auch Cobalt und deren Verbindungen vergleichsweise teuer sind. Es wird ein hoher energetischer und verfahrenstechnischer Aufwand betrieben, um Katalysatorverluste während des Hydroformylierungsprozesses weitestgehend zu vermeiden, beispielsweise durch teils sehr aufwändige Katalysatorrecyclingschritte. Zudem werden Produktaufreinigungsschritte aufwendiger, um sicherzustellen, dass möglichst keine Katalysatorrückstände im Produkt verbleiben.

Weitere Probleme bei den bekannten homogen katalysierten Verfahren sind die Stabilität der Liganden, die die Bedingungen der Hydroformylierung, wie Temperatur, Druck, pH-Wert, usw., überstehen müssen, und der Verbrauch von dem verwendeten Lösemittel während des Prozesses, der durch Nachdosieren ausgeglichen werden muss.

Um die vorgenannten Probleme bei der homogen katalysierten Hydroformylierung zu umgehen, sind Hydroformylierungsverfahren entwickelt worden, bei denen das Katalysatorsystem heterogenisiert wird, insbesondere durch Immobilisierung auf einem Trägermaterial. Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist.

Hydroformylierungsverfahren, bei denen der Katalysator auf einem Trägermaterial heterogenisiert vorkommt, sind beispielsweise in der WO 2015/028284 A1, der EP 3 632 885 A1, EP 3 744 707 A1, der EP 3 632 886 A1 oder der EP 3 736 258 A1 offenbart.

Bei der Durchführung der Hydroformylierung an den heterogenisierten Katalysatoren kann es zu Problemen kommen, wenn Standzeiten z.B. im Rahmen Instandhaltungsarbeiten oder aus anderen produktionstechnischen Gründen auftreten. Standzeit wird im Rahmen der vorliegenden Erfindung so verstanden, dass während dieser Zeit kein Einsatzgemisch durch den Reaktor geleitet werden kann und daher auch keine Hydroformylierungsreaktion stattfinden kann. Nach solch einer Standzeit zeigt die Reaktion meist eine schlechtere Performance, d.h. geringere Umsätze und Selektivitäten. Dies ist vermutlich durch Hochsiederbildung und Zerstörung von Teilen des Katalysators bedingt. Um die Hochsiederbildung zu vermeiden, wird bei Standzeiten das Produktgemisch üblicherweise mit Stickstoff aus dem Reaktionsraum gespült. Hiernach zeigt sich dennoch eine Abnahme der Reaktorperformance.

Die Aufgabe der vorliegenden Erfindung bestand deshalb darin, ein Verfahren zur Hydroformylierung von Olefinen bereitzustellen, welches die vorgenannten Probleme nicht aufweist und insbesondere nach einer Standzeit schneller in Betrieb genommen und ohne Verlust von Katalysatoraktivität gefahren werden kann.

Gelöst wird diese Aufgabe gemäß Anspruch 1 dadurch, dass der Reaktor während der Standzeit mit Synthesegas oder mit Kohlenmonoxid gespült wird. Produktreste werden so aus dem Reaktionsraum gespült und der Katalyatorkomplex durch den hohen CO-partialdruck geschützt.

Ein Gegenstand der vorliegenden Erfindung ist somit ein Verfahren zur Hydroformylierung von C2-bis C8-Olefinen in einer Reaktionszone unter Verwendung eines heterogenisierten Katalysatorsystems, wobei
ein gasförmiges Einsatzgemisch, welches die C2- bis C8-Olefine enthält, zusammen mit Synthesegas in mindestens einem Reaktor über einen in dem mindestens einen Reaktor angeordneten Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, einen Stabilisator und optional eine ionische Flüssigkeit umfasst, heterogenisiert vorliegt, geleitet wird; wobei
der Support ein Monolith, das heißt ein Block aus einem keramischen Material ist, oder in Form eines Pulvers, in Form eines Granulats oder in Form von Formkörpern vorliegt und der Support aus einem carbidischen, nitridischen, silicidischen Material oder Mischungen davon besteht, dadurch gekennzeichnet, dass
es während des Verfahrens zu Standzeiten kommt, in denen kein gasförmiges Einsatzgemisch durch den Reaktor geführt wird, wobei der Reaktor während der Standzeit mit Synthesegas oder mit Kohlenmonoxid gespült wird.

Kennzeichnendes Merkmal der vorliegenden Erfindung ist das Spülen des Reaktors mit Synthesegas und Kohlenmonoxid in der Standzeit. Dadurch lässt sich erreichen, dass nach der Standzeit im Vergleich zu anderen Spülgasen höhere Umsätze und Ausbeuten erhalten werden und der Reaktor demnach schneller wieder im Normalbetrieb gefahren werden kann. Die Temperatur während des Spülens mit Synthesegas oder Kohlenmonoxid liegt vorzugsweise im Bereich von 20 bis 200 °C, weiterhin bevorzugt im Bereich von 22 bis 175 °C und besonders bevorzugt im Bereich von 85 bis 150 °C. Es ist weiterhin bevorzugt, dass der Reaktor in der Standzeit maximal weniger als 10 °C unterhalb der Reaktionstemperatur gehalten wird. Eine Abkühlung soll dadurch vermieden werden. Der Druck während des Spülens ist eher unkritisch, sollte den Druck während der Hydroformylierung nicht überschreiten.

Als Einsatzgemisch können alle Gemische eingesetzt werden, die C2- bis C8-Olefine, vorzugsweise C2- bis C5-Olefine, insbesondere Ethen, Propen, 1-Buten, 2-Buten, 1-Penten oder 2-Penten, als Edukte umfassen. Die Menge an Olefinen in den Einsatzgemischen sollte verständlicherweise hoch genug sein, um eine Hydroformylierungsreaktion wirtschaftlich betreiben zu können. Zu den in dem erfindungsgemäßen Verfahren einsetzbaren Einsatzgemischen gehören insbesondere auch technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.-% Butane, vorzugsweise 60 bis 80 Gew.-% Butane.

Das erfindungsgemäße Verfahren wird in mindestens einem Reaktor, in dem die erfindungsgemäße Hydroformylierung stattfindet, durchgeführt. In dem mindestens einen Reaktor ist der Support mit dem heterogenisierten Katalysatorsystem angeordnet. In einer weiteren Ausführungsform der vorliegenden Erfindung kann das Verfahren auch in mehrere Reaktoren, die parallel oder in Reihe geschaltet vorliegen können, durchgeführt werden. Vorzugsweise sind die Reaktoren in diesem Fall parallel geschaltet und werden alternierend verwendet.

Die Hydroformylierung wird vorzugsweise bei den folgenden Bedingungen durchgeführt: Die Temperatur bei der Hydroformylierung sollte im Bereich von 65 bis 200 °C, vorzugsweise 75 bis 175 °C und besonders bevorzugt 85 bis 150 °C liegen. Die Temperatur kann über einen geeignete Kühlvorrichtung, beispielsweise einen Kühlmantel eingestellt werden. Der Druck sollte 35 bar, vorzugsweise 30 bar, besonders bevorzugt 25 bar während der Hydroformylierung nicht überschreiten. Das molare Verhältnis zwischen Synthesegas und dem Einsatzgemisch sollte zwischen 6:1 und 1:1, vorzugsweise zwischen 5:1 und 3:1 liegen. Optional kann das Einsatzgemisch mit Inertgas verdünnt werden, beispielsweise mit den in technischen Kohlenwasserstoffströmen befindlichen Alkanen.

Das bei dem erfindungsgemäßen Hydroformylierungsverfahren eingesetzte Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, weiterhin bevorzugt Cobalt und Rhodium, besonders bevorzugt Rhodium, mindestens einen organischen Phosphor-enthaltenden Liganden und einen Stabilisator.

Der Stabilisator ist vorzugsweise eine organische Aminverbindung, besonders bevorzugt eine organische Aminverbindung, die mindestens eine 2,2,6,6-Tetramethylpiperidineinheit nach Formel (I) enthält:

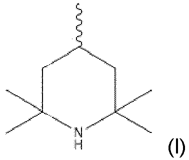

In einer besonders bevorzugten Ausführungsform der vorliegenden Erfindung ist der Stabilisator ausgewählt aus der Gruppe, bestehend aus den Verbindungen der nachfolgenden Formeln (I.1), (I.2), (I.3), (I.4), (I.5), (I.6), (I.7) und (I.8). 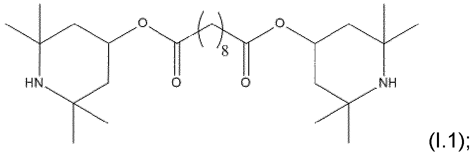 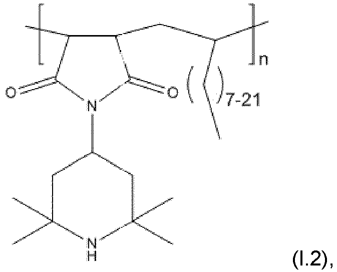
wobei n einer ganzen Zahl von 1 bis 20 entspricht; 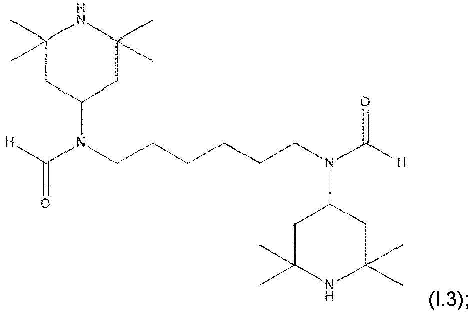 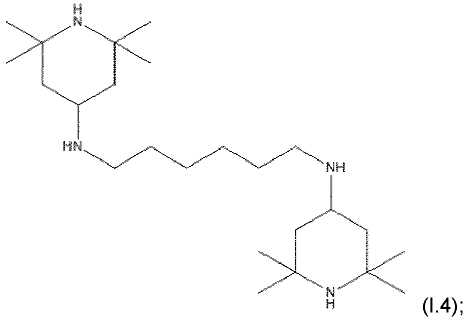 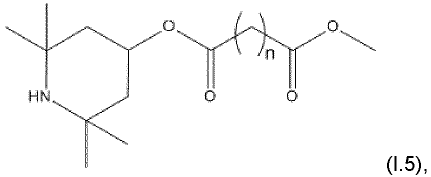
wobei n einer ganzen Zahl von 1 bis 12 entspricht; 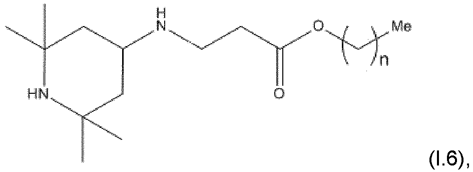
wobei n einer ganzen Zahl von 1 bis 17 entspricht; 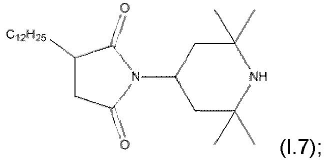 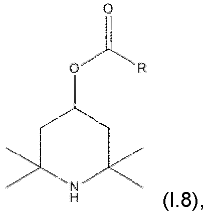
wobei R einer C6- bis C20-Alkylgruppe entspricht.

Für alle filmbildenden Komponenten, d. h. in diesem Fall der Stabilisator, sollte die Gaslöslichkeit für die Reaktanden besser sein als die Gaslöslichkeit der Produkte. Bereits dadurch kann eine partielle Stofftrennung zwischen eingesetzten Eduktolefinen und gebildeten Produktaldehyden erreicht werden. Grundsätzlich wären dafür auch andere filmbildende Substanzen denkbar, allerdings ist darauf zu achten, dass es nicht zu einer erhöhten Hochsiederbildung kommt und/oder die Nachführung der Eduktolefine eingeschränkt wird.

Der organische Phosphor-enthaltende Ligand für das erfindungsgemäße Katalysatorsystem kann aus den für die Hydroformylierungen bekannten Liganden ausgewählt werden. Eine Vielzahl geeigneter Liganden ist dem Fachmann aus der Patent- und Fachliteratur bekannt, beispielsweise Mono- oder Biphosphitliganden. Der organische Phosphor-enthaltende Ligand weist vorzugsweise gemäß der allgemeinen Formel (II) eine Biphosphitstruktur auf

R' - A - R" - A - R‴ (II)

wobei R', R" und R‴ jeweils organische Reste sind und beide A jeweils eine brückende -O-P(-O)₂-Gruppe sind, wobei zwei der drei Sauerstoffatome -O- jeweils an Rest R' und den Rest R‴ gebunden sind, mit der Maßgabe das R' und R‴ nicht identisch sind. Die organischen Reste R', R" und R‴ enthalten vorzugsweise keine endständige Trialkoxysilangruppe.

In einer bevorzugten Ausführungsform sind R', R" und R‴ in der Verbindung der Formel (VI), vorzugsweise ausgewählt aus substituierten oder unsubstituierten 1,1'-Biphenyl-, 1,1'-Binaphthyl- und ortho-Phenylgruppen, insbesondere aus substituierten oder unsubstituierten 1,1'-Biphenylgruppen, mit der Maßgabe das R' und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Das vorgenannte Katalysatorsystem liegt erfindungsgemäß heterogenisiert auf dem Support aus einem porösen keramischen Material vor. Im Sinne der vorliegenden Erfindung ist der Ausdruck "heterogenisiert auf einem Support" so zu verstehen, dass das Katalysatorsystem durch Ausbildung eines dünnen, festen oder flüssigen Films mithilfe des Stabilisators auf der inneren und/oder äußeren Oberfläche des Supports immobilisiert wird. Der Film kann auch bei Raumtemperatur fest und bei Reaktionsbedingungen flüssig sein.

Die innere Oberfläche des festen Trägermaterials umfasst insbesondere die innere Oberfläche der Poren. Immobilisierung umfasst begrifflich sowohl den Fall, dass das Katalysatorsystem und/oder die katalytisch aktive Spezies gelöst in dem festen oder flüssigen Film vorliegen, als auch die Fälle, dass der Stabilisator als Haftvermittler wirkt oder dass das Katalysatorsystem auf der Oberfläche adsorbiert wird, nicht jedoch chemisch bzw. kovalent gebunden auf der Oberfläche vorliegt.

Es liegt erfindungsgemäß also keine Reaktionslösung im klassischen Sinne vor, in der der Katalysator homogen gelöst ist, sondern das Katalysatorsystem befindet sich dispers verteilt auf der Oberfläche und/oder in den Poren des Supports.

Das poröse Supportmaterial ist vorzugsweise ausgewählt aus der Gruppe, bestehend aus einer nitridischen Keramik, einer carbidischen Keramik, einer silicidischen Keramik und Mischungen davon, beispielsweise carbonitridische Materialien.

Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik, besonders bevorzugt aus Siliciumcarbid.

Der Support kann im vorliegenden Fall als Monolith, das heißt einem Block aus einem keramischen Material, oder in Form eines Pulvers, in Form eines Granulats oder in Form von Formkörpern vorliegen.

Ist der Support ein Monolith, besteht der Support aus einem Block (ein dreidimensionales Objekt) aus dem porösen keramischen Material. Der Block kann sowohl einstückig ausgebildet sein als auch aus mehreren, also mindestens zwei Einzelteilen bestehen, die zu dem Block zusammengefügt werden können und/oder miteinander fest oder lösbar verbunden sind.

Der Support aus dem porösen keramischen Material ist vorzugsweise ein sich dreidimensional erstreckendes Bauteil, welches in seinem Querschnitt grundsätzlich beliebige geometrische Formen, beispielsweise rund, eckig, quadratisch o. ä., aufweisen kann. Das sich dreidimensional erstreckende Bauteil, welches als Support eingesetzt werden kann, weist in einer bevorzugten Ausführungsform eine Längsrichtung (Richtung der längsten Ausdehnung) in Hauptdurchströmungsrichtung (Richtung in die das Einsatzgemisch und das Synthesegas von Einlass zum Auslass des Reaktors strömen) auf.

Der so geformte Supportmonolith aus dem porösen keramischen Material weist zumindest einen durchgängigen Kanal in Hauptdurchströmungsrichtung auf. Der oder die Kanäle können jedoch auch so ausgestaltet sein, dass sie nicht komplett durchgängig sind, sondern zu dem Ende, welches dem Einlass des Reaktors gegenüberliegt, einen Abschluss aufweisen bzw. der Kanal zu diesem Ende hin geschlossen ist. Der Supportmonolith kann auch mindestens zwei oder mehr Kanäle aufweisen. Der Durchmesser der Kanäle kann im Bereich von 0,25 bis 50 mm, vorzugsweise im Bereich von 1 bis 30 mm, weiterhin bevorzugt im Bereich von 1,5 bis 20 mm und besonders bevorzugt im Bereich von 2 bis16 mm liegen. Sind mehrere Kanäle vorhanden, können die Durchmesser der Kanäle gleich oder verschieden voneinander sein. Der Durchmesser der Kanäle ist im Vergleich zu dem oder zu einem der Durchmesser des gesamten Supports insbesondere so zu wählen, dass die mechanische Stabilität nicht beeinträchtigt wird.

Darüber hinaus ist der Supportmonolith aus dem keramischen Material porös, weist also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

In einer bevorzugten Ausführungsform weist der Supportmonolith zumindest teilweise durchgängige Poren auf, die sich von der Oberfläche zu den Kanälen hin und/oder von einem Kanal zu dem oder zu den nächstliegenden Kanälen erstrecken. Möglich ist auch das mehrere Poren miteinander verbunden sind und so insgesamt eine einzige durchgängige Pore bilden.

Der Support kann erfindungsgemäß auch in Form eines Pulvers, in Form eines Granulats oder in Form von Formkörpern wie Pellets, Ringe, Kugeln o.ä. vorliegen. Der mittlere Partikeldurchmesser (d50) des Supports kann von 0,1 mm bis 7 mm, vorzugsweise 0,3 bis 6 mm, besonders bevorzugt von 0,5 mm bis 5 mm betragen. Der mittlere Partikeldurchmesser kann mittels bildgebender Verfahren ermittelt werden, insbesondere durch die in den Normen ISO 13322-1 (Stand: 2004-12-01) und ISO 13322-2 (Stand: 2006-11-01) genannten Verfahren ermittelt werden. Die Herstellung des Supports in Form eines Pulvers, in Form eines Granulats oder in Form von Formkörpern kann nach dem Fachmann bekannten Verfahren erfolgen. Beispielsweise könnte es dadurch erfolgen, dass ein Monolith aus dem carbidischen, nitridischen, silicidischen Material oder Mischungen davon mechanisch zerkleinert wird, beispielsweise mit einem Backenbrecher, und die Partikelgröße des erhaltenen Bruchgranulats mittels Sieben eingestellt wird.

Enstsprechend dem Supportmonolith sind die Partikel des Pulvers, des Granulats oder die Formkörper aus dem keramischen Material porös, weisen also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

Die Herstellung des Supports, egal ob Monolith, Pulver, Granulat oder Formkörper, erfolgt wie nachfolgend beschrieben:
Auf den bereitgestellten pulver-, granulat- oder pelletförmigen Support aus dem keramischen Material kann zusätzlich ein sogenannter Washcoat aufgetragen werden, welcher bezogen auf das keramische Material des Supports aus dem gleichen oder einem unterschiedlichen Keramikmaterial, vorzugsweise Siliciumoxid. Der Washcoat selbst kann porös oder unporös sein, vorzugsweise ist der Waschcoat unporös. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports. In einer bevorzugten Ausführungsform der vorliegenden Erfindung weist der Support aber keinen Washcoat auf.

Auf den Support mit oder ohne Washcoat wird das Katalysatorsystem aufgebracht. Dazu wird zunächst eine Katalysatorlösung durch Mischen, insbesondere bei Raumtemperatur und Umgebungsdruck, hergestellt, wobei die Katalysatorlösung mindestens einen organischen Phosphor-haltigen Ligand, mindestens einen Metall-Precursor, beispielsweise Chloride, Oxide, Carboxylate des jeweiligen Metalls, mindestens einen Stabilisator und mindestens ein Lösemittel umfasst. Optional kann bei der Herstellung des Katalysatorsystems eine ionische Flüssigkeit verwendet werden, die Katalysatorlösung kann aber auch explizit ohne ionische Flüssigkeit angesetzt werden. Die Herstellung der Katalysatorlösung sollte insbesondere in einer inerten Umgebung, bspw. einer Glovebox erfolgen. Inerte Umgebung heißt in diesem Fall eine möglichst Wasser- und Sauerstoff-freie Atmosphäre.

Das Lösemittel kann aus allen Lösemittelklassen gewählt werden (protisch, aprotisch, polar oder unpolar) Voraussetzung für das Lösemittel ist die Löslichkeit von Katalysatorsystem (Ligand, Metall Precursor, Stabilisator und optional die ionische Flüssigkeit) und bevorzugt auch der bei der Hydroformylierung entstehenden Hochsieder. Die Löslichkeit kann innerhalb des Immobilisierungsschrittes durch Aufheizen erhöht werden.

Das Lösemittel ist vorzugsweise aprotisch, polar, wie z. B. Acetonitril und Ethylacetat oder aber auch aprotisch, unpolar wie z. B. THF und Dietehylether. Auch Chlorkohlenwasserstoffe wie z. B. Dichlormethan oder Aldehyde können als Lösemittel verwendet werden.

Die so hergestellte Katalysatorlösung wird dann mit dem Support (optional inkl. Washcoat) in Kontakt gebracht, beispielsweise durch Eintauchen (Dip-Coating) oder durch Befüllen eines Druckgefäßes, beispielsweise direkt im Reaktor (in-situ-Imprägnierung). Sofern das Aufbringen der Katalysatorlösung außerhalb des Reaktors erfolgt, muss der Support nach Abtrennen des Lösemittels natürlich wieder in den Reaktor eingebaut werden. Bevorzugt wird die Katalysatorlösung direkt im Reaktor auf den Support mit dem Washcoat aufgebracht, weil dadurch möglicherweise zeitaufwändige Ein- und Ausbauschritte sowie eine mögliche Kontamination des Katalysators vermieden werden können.

Das Befüllen des Reaktors mit der Katalysatorlösung kann über die normalen Ein- bzw. Ausgänge erfolgen, beispielsweise mittels einer Pumpe. Flüssigkeitsverteiler oder Düsen innerhalb des Reaktors können für eine gleichmäßige Verteilung der Katalysatorflüssigkeit sorgen, ebenso wie optional vorliegende Druckverlusteinbauten oder Regelungen für die Dosiergeschwindigkeit.

Nach dem Aufbringen des Katalysatorsystems wird das Lösemittel abgetrennt. Dabei wird zunächst die restliche Katalysatorlösung über den Auslass des Reaktors abgelassen. Danach werden im Reaktor verbliebene Lösemittelreste durch Einstellen des Drucks oder Erhöhung der Temperatur verdampft. Die Einstellung des Drucks kann in einer anderen Ausführungsform auch unter gleichzeitiger Erhöhung der Temperatur durchgeführt werden. Die Temperatur kann in Abhängigkeit vom Lösemittel 20 bis 150 °C betragen. Der Druck kann in Abhängigkeit vom Lösemittel auf ein Hochvakuum (10⁻³ bis 10⁻⁷ mbar) eingestellt werden, je nach Lösemittel und Temperatur sind aber auch Überdrücke von einigen mbar bis zu mehreren bar denkbar.

Der Stabilisator und die optional vorhandene ionische Flüssigkeit verbleiben mit dem Katalysator aus dem Übergangsmetall, insbesondere Cobalt oder Rhodium, und dem organischen Phosphor-haltigen Liganden heterogenisiert auf dem Support.

Das Aufbringen des Katalysatorsystem auf den Support kann sowohl direkt im Reaktor (in situ) oder außerhalb des Reaktors erfolgen. Wird das Katalysatorsystem außerhalb des Reaktors aufgebracht, sollte der Support immer unter Luftabschluss transportiert werden muss, was beispielsweise mit einem Stickstoffgegenstrom realisiert werden. Das Aufbringen des Katalysatorsystems wird in einer bevorzugten Ausführungsform der vorliegenden Erfindung direkt im Reaktor, also in situ, aufgebracht. Nach dem Abtrennen des Lösemittels kann der Reaktor sofort eingesetzt werden und mit dem Einsatzgemisch beschickt werden. Das hat den Vorteil, dass keine zeitaufwändigen Ein- und Ausbauschritte notwendig sind, die einen längeren Ausfall des Reaktors zur Folge hätten. Zudem ist die Größe des Supports dann nicht mehr dadurch limitiert, dass geeignete Räumlichkeiten mit inerten Umgebungen einer bestimmten Größe vorliegen. Die Größe des Supports kann in Abhängigkeit vom Reaktordesign frei gewählt werden.

Nach erfolgtem Aufbringen des Katalysatorsystems auf den Support und erfolgter Abtrennung des Lösemittels kann die Anlage, insbesondere der Reaktor durch eine zwei- oder mehrstufige Anfahrprozedur hochgefahren werden, also in den Betrieb überführt werden. Eine geeignete Anfahrprozedur ist beispielsweise in der EP 3 632 887 beschrieben.

Aus der Reaktionszone, in der die erfindungsgemäße Hydroformylierung durchgeführt wird, wird vorzugsweise kontinuierlich ein gasförmiger Austrag entnommen, der mindestens einen Teil der entstandenen Produktaldehyde und mindestens einen Teil der nicht umgesetzten Olefine enthält. Der gasförmige Austrag kann einem oder mehreren Stofftrennungsschritt(en) unterworfen werden, bei der der gasförmige Austrag in mindestens eine an nicht umgesetzten Olefinen-reiche Phase und mindestens eine Produktaldehyd-reiche Phase getrennt wird.

Die Stofftrennnung kann mit bekannten Stofftrennungsverfahren, wie Kondensation, Destillation, Zentrifugation, Nanofiltration oder Kombination von mehreren davon, vorzugsweise Kondensation oder Destillation, durchgeführt werden.

Im Falle einer mehrstufigen Stofftrennung kann die bei der ersten Stofftrennung gebildete Produktaldehyd-reiche Phase einer zweiten Stofftrennung, insbesondere einer nachfolgenden Aldehydabtrennung zugeführt werden, bei der das Produktaldehyd von den anderen in dieser Phase befindlichen Stoffen, häufig Alkane und Eduktolefine, abgetrennt wird. Die an nicht umgesetzten Olefin-reiche Phase kann zu dem Hydroformylierungsschritt oder in Falle einer mehrstufigen Ausgestaltung zu einer der Hydroformylierungsschritten zurückgeführt werden, um die darin enthaltenen Olefine auch noch zum Produktaldehyd zu hydroformylieren.

Bei der Stofftrennung kann neben den genannten Phasen auch ein Purgegasstrom entnommen werden, der eine zur an nicht umgesetzten Olefin-reiche Phase zumindest ähnliche oder identische Zusammensetzung aufweist. Der Purgegasstrom kann ebenfalls zur zweiten Stofftrennung bzw. Aldehydabtrennung geleitet werden, um die darin enthaltenen Produktaldehyde abzutrennen und um Verunreinigungen (z. B. Stickstoff im Synthesegas) oder inerte Substanzen (z. B. Alkane im Einsatzgemisch) aus dem System auszutragen. Die Verunreinigungen oder inerte Substanzen können üblicherweise bei der zweiten Stofftrennung als leichtflüchtige Stoffe, beispielsweise am Kopf einer Kolonne, abgenommen werden.

Ein weiterer Gegenstand der vorliegenden Erfindung ist auch eine Anlage mit der das vorliegende Verfahren durchgeführt werden kann und welches insbesondere einen Reaktor, in dem der erfindungsgemäße Hydroformylierungsschritt durchgeführt wird umfasst. Zusätzlich kann die Anlage eine Stofftrennungseinheit umfassen, mit der der gasförmige Austrag des Hydroformylierungsschritts in mindestens eine an nicht umgesetzten Olefin reiche Phase und mindestens eine Produktaldehyd-reiche Phase getrennt wird, wobei diese Stofftrennungseinheit nach der erfindungsgemäßen Hydroformylierung angeordnet ist. Stromabwärts der ersten Stofftrennung kann eine zweite Stofftrennungseinheit, insbesondere eine Aldehydabtrennungseinheit, vorliegen, mit der der Produktaldehyd abgetrennt wird.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

### Beispiel:

### Versuche 1 - 4: Spülen mit unterschiedlichen Gasen während Standzeiten des Systems

Als Ausgangsmaterial für den Support wurden SiC-Pellets verwendet (SIKAT SarL SIC-3), Die SiC-Pellets wurden in eine 20 cm lange runde Reaktorhülse mit einem Durchmesser von einem Zoll (ca. 2,54 cm) eingebracht, wobei ober- und unterhalb des Granulats Glasperlen ähnlicher Größe eingefüllt wurden. Die SiC-Pellets wurde dann mit einer Katalysatorlösung, enthaltend Rh(acac)(CO)₂, Bisphephos (Ligand), Bis(2,2,6,6-tetramethyl-4-piperidyl)sebacat (Stabilisator) und Dichlormethan als Lösemittel beaufschlagt. Dazu wurde die Katalysatorlösung nach dem Spülen des Reaktors mit Stickstoff mit leichtem Überdruck in den Reaktor eingeleitet. Nach dem Entfernen des Lösemittels aus dem Reaktor durch Ablassen und Verdampfen wurde das auf dem Supportgranulat heterogenisierte Katalysatorsystem zur Hydroformylierung verwendet.

Als Einsatzgemisch wurde ein Kohlenwasserstoffstrom mit der folgenden Zusammensetzung verwendet:

| | Menge (Gew.-%) |
|---|---|
| Butene | 13 |
| Butane | 87 |

Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 - 130 °C und einem Druck von 17 bar durchgeführt.

Während des Versuchs wurde der Strom der Reaktanden für mehrere längere Zeiträume unterbrochen (Standzeit) und verschiedene Strategien zur Aktivitätserhaltung des Katalysatorsystems erprobt.

Versuch 1: In der ersten Standzeit 1 wurde der weiterhin auf 120 °C erwärmte Reaktor mit einem Stickstoffstrom bei einem Druck von 1,2 bar gespült.

Versuch 2: In der zweiten Standzeit 2 wurde der weiterhin auf 120 °C erwärmte Reaktor mit einem Stickstoffstrom bei einem Druck von 17 bar gespült.

Versuch 3: In der dritten Standzeit 3 wurde der weiterhin auf 120 °C erwärmte Reaktor mit Synthesegas bei einem Druck von 17 bar gespült.

Versuch 4: In der vierten Standzeit 4 wurde der weiterhin auf 120 °C erwärmte Reaktor mit Synthesegas bei einem Druck von 1,2 bar gespült.

Vor und nach den jeweiligen Versuchsdaten wurden die Umsätze und Ausbeuten bestimmt. Die Ergebnisse sind in der nachfolgenden Tabelle 1 gezeigt:

**Tabelle 1: Ergebnisse der oben beschriebenen Versuche**

| Versuch | | Ausbeute | Umsatz |
|---|---|---|---|
| Versuch 1 | Vorher | 47,5% | 52,7% |
| | Nachher | 7,4% | 26,7% |
| Versuch 2 | Vorher | 24,5% | 45,8% |
| | Nachher | 11% | 30,8% |
| Versuch 3 | Vorher | 38,6% | 54,7% |
| | Nachher | 44% | 60,3% |
| Versuch 4 | Vorher | 44,9% | 61,7% |
| | Nachher | 44,1% | 60,9% |

Es zeigt sich, dass bei einem Spülen mit Synthesegas die Ausbeuten und Umsätze nach der Standzeit wieder auf einem ähnlichen hohen Wert sind. Bei Spülen mit Stickstoff sind Ausbeuten und Umsätze deutlich geringer als vor der Standzeit.

## Patentansprüche

1. Verfahren zur Hydroformylierung von C2- bis C8-Olefinen in einer Reaktionszone unter Verwendung eines heterogenisierten Katalysatorsystems, wobei
ein gasförmiges Einsatzgemisch, welches die C2- bis C8-Olefine enthält, zusammen mit Synthesegas in mindestens einem Reaktor über einen in dem mindestens einen Reaktor angeordneten Support aus einem porösen keramischen Material, auf dem das Katalysatorsystem, welches ein Metall aus der 8. oder 9. Gruppe des Periodensystems der Elemente, mindestens einen organischen Phosphor-enthaltenden Liganden, einen Stabilisator umfasst, heterogenisiert vorliegt, geleitet wird; wobei
der Support ein Monolith, das heißt ein Block aus einem keramischen Material ist, oder in Form eines Pulvers, in Form eines Granulats oder in Form von Formkörpern vorliegt und der Support aus einem carbidischen, nitridischen, silicidischen Material oder Mischungen davon besteht, **dadurch gekennzeichnet, dass**
es während des Verfahrens zu Standzeiten kommt, in denen kein gasförmiges Einsatzgemisch durch den Reaktor geführt wird, wobei der Reaktor während der Standzeit mit Synthesegas oder mit Kohlenmonoxid gespült wird.

2. Verfahren nach Anspruch 1, wobei auf den Support kein Washcoat aufgetragen wird, sondern der Support ohne Washcoat eingesetzt werden.

3. Verfahren nach Anspruch 1 oder 2, wobei der organische Phosphor-enthaltende Ligand des Hydroformylierungskatalysatorsystems vorzugsweise die allgemeine Formel (II)
R' - A - R" - A - R‴ (II)
aufweist, wobei R', R" und R‴ jeweils organische Reste sind, mit der Maßgabe das R' und R‴ nicht identisch sind, und beide A jeweils eine brückende -O-P(-O)₂-Gruppe sind, wobei zwei der drei Sauerstoffatome -O- jeweils an Rest R' und den Rest R‴ gebunden sind.

4. Verfahren nach einem der Ansprüche 1 bis 3, wobei der Stabilisator eine organische Aminverbindung ist, die mindestens eine 2,2,6,6-Tetramethylpiperidineinheit nach Formel (I) enthält:

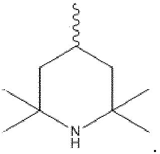

5. Verfahren nach einem der Ansprüche 1 bis 4, wobei die nitridische Keramik ausgewählt wird aus Siliciumnitrid, Bornitrid, Aluminiumnitrid und Mischungen davon; die carbidische Keramik ausgewählt wird aus Siliciumcarbid, Borcarbid, Wolframcarbid oder Mischungen davon; und die silicidische Keramik Molbydänsilicid ist.

6. Verfahren nach Anspruch 5, wobei der Support aus einer carbidische Keramik besteht.

7. Verfahren nach Anspruch 6, wobei der Support aus Siliciumcarbid besteht.

8. Verfahren nach einem der Ansprüche 1 bis 7, wobei die Hydroformylierung bei einer Temperatur im Bereich von 65 bis 200 °C, vorzugsweise 75 bis 175 °C und besonders bevorzugt 85 bis 150 °C, durchgeführt wird.

9. Verfahren nach einem der Ansprüche 1 bis 8, wobei der Druck bei der Hydroformylierung nicht größer als 35 bar, vorzugsweise nicht größer als 30 bar, besonders bevorzugt nicht größer als 25 bar ist.

10. Verfahren nach einem der Ansprüche 1 bis 9, wobei das Katalysatorsystem keine ionische Flüssigkeit umfasst.

11. Verfahren nach einem der Ansprüche 1 bis 10, wobei bei dem Verfahren zur Hydroformylierung C4-Olefine eingesetzt werden.

12. Verfahren nach einem der Ansprüche 1 bis 11, wobei als ein Metall aus der 8. oder 9. Gruppe des Periodensystems der Elemente Rhodium eingesetzt wird.

## Claims

1. Process for hydroformylating C2 to C8 olefins in a reaction zone using a heterogenized catalyst system, wherein
a gaseous feed mixture containing the C2 to C8 olefins is passed together with synthesis gas in at least one reactor over a support arranged in the at least one reactor that is composed of a porous ceramic material on which the catalyst system, which comprises a metal from group 8 or group 9 of the periodic table of the elements, at least one organic phosphorus-containing ligand and a stabilizer, is present in a heterogenized form; wherein the support is a monolith, i.e. a block of a ceramic material, or is present in the form of a powder, in the form of a granular material or in the form of shaped bodies and the support consists of a carbidic, nitridic, silicidic material or mixtures thereof, **characterized in that**
downtimes occur during the process in which no gaseous feed mixture is passed through the reactor, the reactor being purged during the downtime with synthesis gas or carbon monoxide.

2. Process according to Claim 1, wherein the support does not have a washcoat applied, but is used without washcoat.

3. Process according to Claim 1 or 2, wherein the organic phosphorus-containing ligand of the hydroformylation catalyst system preferably has the general formula (II)
R' - A - R" - A - R‴ (II)
where R', R'' and R‴ are each organic radicals, with the proviso that R' and R''' are nonidentical, and the two A are each a bridging -O-P(-O)₂ group, wherein two of the three oxygen atoms -O- are attached respectively to the radical R' and to the radical R‴.

4. Process according to any of Claims 1 to 3, wherein the stabilizer is an organic amine compound containing at least one 2,2,6,6-tetramethylpiperidine unit of formula (I):

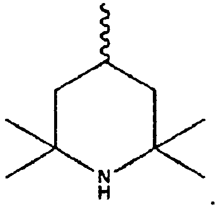

5. Process according to any of Claims 1 to 4, wherein the nitridic ceramic is selected from silicon nitride, boron nitride, aluminium nitride and mixtures thereof; the carbidic ceramic is selected from silicon carbide, boron carbide, tungsten carbide or mixtures thereof; and the silicidic ceramic is molybdenum silicide.

6. Process according to Claim 5, wherein the support consists of a carbidic ceramic.

7. Process according to Claim 6, wherein the support consists of silicon carbide.

8. Process according to any of Claims 1 to 7, wherein the hydroformylation is carried out at a temperature in the range from 65 to 200°C, preferably 75 to 175°C and more preferably 85 to 150°C.

9. Process according to any of Claims 1 to 8, wherein the pressure in the hydroformylation is not greater than 35 bar, preferably not greater than 30 bar, more preferably not greater than 25 bar.

10. Process according to any of Claims 1 to 9, wherein the catalyst system does not contain any ionic liquid.

11. Process according to any of Claims 1 to 10, wherein C4 olefins are used in the hydroformylation process.

12. Process according to any of Claims 1 to 11, wherein the employed metal from group 8 or 9 of the periodic table of the elements is rhodium.

## Revendications

1. Procédé d'hydroformylation d'oléfines en C2-C8 dans une zone de réaction à l'aide d'un système catalytique hétérogénéisé, dans lequel
un mélange de départ gazeux, qui contient les oléfines en C2-C8, est guidé conjointement avec un gaz de synthèse dans au moins un réacteur sur un support, agencé dans ledit au moins un réacteur et constitué par un matériau céramique poreux, sur lequel se trouve, sous forme hétérogénéisée, le système catalytique, qui comprend un métal du 8e ou 9e groupe du système périodique des éléments, au moins un ligand organique contenant du phosphore, un stabilisant ;
le support étant un monolithe, c'est-à-dure un bloc en un matériau céramique, ou se trouvant sous forme d'une poudre, d'un granulat ou de corps façonnés et le support étant constitué d'un matériau à base de carbure, de nitrure ou de siliciure ou des mélanges de ceux-ci, **caractérisé en ce que**
pendant le procédé, il se produit des durées d'arrêt pendant lesquelles aucun mélange de départ gazeux n'est guidé à travers le réacteur, le réacteur étant rincé par du gaz de synthèse ou du monoxyde de carbone pendant le temps d'arrêt.

2. Procédé selon la revendication 1, aucune couche d'imprégnation n'étant appliquée sur le support, mais le support étant utilisé sans couche d'imprégnation.

3. Procédé selon la revendication 1 ou 2, le ligand organique contenant du phosphore du système catalytique d'hydroformylation présentant de préférence la formule générale (II)
R' - A - R" - A - R‴ (II)
dans laquelle R', R" et R"' représentent à chaque fois des radicaux organiques, étant entendu que R' et R"' ne sont pas identiques et que les deux A représentent à chaque fois un groupe pontant -O-P(-O)₂, deux des trois atomes d'oxygène -O- étant à chaque fois liés au radical R' et au radical R"'.

4. Procédé selon la revendication 1 à 3, le stabilisant étant un composé de type amine organique qui contient au moins un motif 2,2,6,6-tétraméthylpipéridine selon la formule (I) :

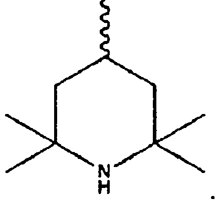

5. Procédé selon l'une des revendications 1 à 4, la céramique à base de nitrure étant choisie parmi le nitrure de silicium, le nitrure de bore, le nitrure d'aluminium et leurs mélanges ; la céramique à base de carbure étant choisie parmi le carbure de silicium, le carbure de bore, le carbure de tungstène ou leurs mélanges ; et la céramique à base de siliciure étant le siliciure de molybdène.

6. Procédé selon la revendication 5, le support étant constitué d'une céramique à base de carbure.

7. Procédé selon la revendication 6, le support étant constitué de carbure de silicium.

8. Procédé selon l'une des revendications 1 à 7, l'hydroformylation étant réalisée à une température dans la plage de 65 à 200°C, de préférence de 75 à 175°C et de manière particulièrement préférée de 85 à 150°C.

9. Procédé selon l'une des revendications 1 à 8, la pression lors de l'hydroformylation n'étant pas supérieure à 35 bars, de préférence pas supérieure à 30 bars, de manière particulièrement préférée pas supérieure à 25 bars.

10. Procédé selon l'une des revendications 1 à 9, le système catalytique ne comprenant pas de liquide ionique.

11. Procédé selon l'une des revendications 1 à 10, des oléfines en C4 étant utilisées lors du procédé d'hydroformylation.

12. Procédé selon l'une des revendications 1 à 11, du rhodium étant utilisé comme métal du 8e ou 9e groupe du système périodique des éléments.
